# FASCICULE DE BREVET EUROPEEN

(11) **EP 3 322 347 B1**
(45) Date de publication et mention de la délivrance du brevet: **13.05.2020**
(21) Numéro de dépôt: 16750948.8
(22) Date de dépôt: 12.07.2016
(51) Int. Cl.: A61B 10/02, A61B 17/34, A61B 34/20, A61B 34/10, A61B 17/00, A61B 90/00, A61B 18/00, A61N 5/10

(54) **SYSTEME DE GUIDAGE AUTONOME D'UN EQUIPEMENT PORTE-AIGUILLE**
AUTONOMES FÜHRUNGSSYSTEM FÜR NADELHALTEAUSRÜSTUNG
AUTONOMOUS GUIDANCE SYSTEM FOR NEEDLE-HOLDING EQUIPMENT

(30) Priorité: 16.07.2015 FR 1556702
(43) Date de publication de la demande: 23.05.2018
(73) Titulaire: Université de Lille, 59800 Lille (FR); Centre National de la Recherche Scientifique (CNRS), 75016 Paris (FR); Centre Oscar Lambret, 59000 Lille (FR)
(72) Inventeur: MERZOUKI, Rochdi, 59650 Villeneuve d'Ascq (FR); COELEN, Vincent, 59110 La Madeleine (FR); LARTIGAU, Eric, 59110, La Madeleine (FR); BELAROUCI, Abdelkader, 59100 Roubaix (FR)
(74) Mandataire: Plasseraud IP
(86) Numéro de dépôt international: PCT/FR2016/051783
(87) Numéro de publication internationale: WO 2017/009572

(56) Documents cités:
- US-A- 5 540 649
- US-A1- 2003 018 232
- US-A1- 2003 135 115
- US-A1- 2003 229 282
- US-A1- 2009 030 339
- US-A1- 2009 093 715
- US-A1- 2015 190 653

## Description

### Domaine technique et art antérieur

La présente invention concerne le domaine des équipements de traitement et/ou de diagnostic des pathologies d'un organe tel que par exemple la prostate.

La présente invention porte plus précisément sur un système robotisé pour le guidage autonome d'un équipement porte-aiguille ou porte-seringue pour un traitement interne (curiethérapie et/ou biopsie) d'au moins une tumeur localisée sur un organe.

Un des objets de la présente invention est de contrôler le guidage d'une aiguille et son insertion dans un tel organe selon une direction et une profondeur déterminée pour atteindre au moins une cible.

Un des autres objets de la présente invention est de contrôler le dépôt d'éléments radioactifs d'une aiguille lors de son insertion dans un tel organe selon une direction désirée.

Le cancer de la prostate concerne environ un homme sur six entre 60 et 79 ans.

Il est donc important de mettre en place des techniques efficaces de traitement et de diagnostic pour limiter le nombre de décès liés à ce genre de pathologies.

Pour traiter le cancer de la prostate, on utilise la curiethérapie.

La curiethérapie est une technique de radiothérapie interne visant à irradier la prostate en plaçant dans la prostate des radio-isotopes émetteurs de rayonnements ionisants de basse énergie (I125).

Les études cliniques ont montré que cette technique présente un bon taux de rémission ; cette technique est par ailleurs relativement bien tolérée par le patient.

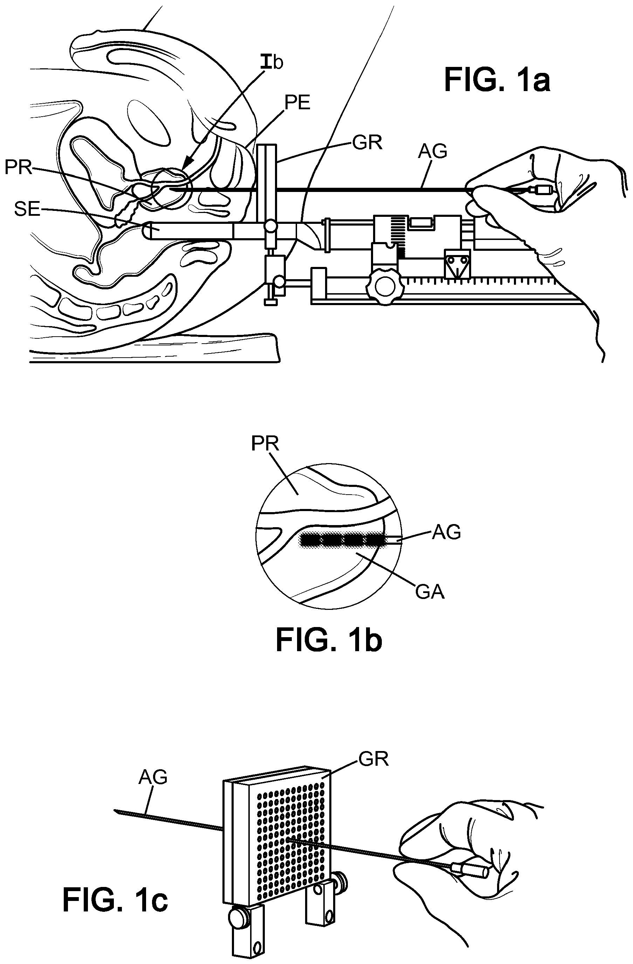

Le dispositif médical classiquement utilisé pour la curiethérapie est illustré ici dans les figures 1a, 1b et 1c qui sont annexées à la présente description.

Il comporte une sonde échographique endo-rectale SE couplée à un module ultrason permettant de balayer l'ensemble de la prostate PR par des ultrasons.

Comme illustré notamment en figure la et 1c, le dispositif médical utilisé comporte également une grille de repérage GR posée contre le périnée PE pour permettre le repérage de l'implantation des aiguilles AG, conformément aux images échographiques et à la dosimétrie prévue.

Le principe de cette technique consiste donc à insérer successivement, sous guidage échographique, une pluralité d'aiguilles AG chargées de radio-isotopes GA à travers une grille GR homogène placée contre le périnée PE du patient.

Les aiguilles AG sont tout d'abord chargées en radio-isotopes sur la base d'un dosage déterminé par le biais d'une imagerie de référence de la prostate PR.

Les émetteurs ionisants ou radio-isotopes GA doivent ensuite être placés en trains rectilignes de grains dans la prostate PR, ceci avec une précision millimétrique. En effet, l'erreur de placement tolérée ne doit pas dépasser plus de 5 millimètres autour de la cible.

Lors d'une séance de curiethérapie, le patient est sous anesthésie générale et la manipulation des aiguilles pré-chargées se fait manuellement par le chirurgien (voir figure 1c).

Cette intervention chirurgicale est longue ; elle dure environ 1 heure et 30 minutes.

Les gestes à exécuter sont très techniques ; ils nécessitent beaucoup de concentration, de pratique et d'entraînement.

Lorsque le chirurgien manque sa cible (il peut contrôler ses gestes sur un écran de contrôle), il doit recommencer l'insertion de l'aiguille autant de fois que nécessaire.

Le Demandeur soumet que les nombreuses insertions d'aiguilles dans le corps du patient entraînent bien souvent un risque élevé d'œdèmes externes, ce qui provoque des douleurs, voire des complications, dans la phase post-opératoire.

Corrélativement, pour diagnostiquer le cancer de la prostate, on procède généralement à une biopsie.

Une biopsie de la prostate consiste à prélever un morceau de la prostate à l'aide d'une aiguille qu'on introduit dans la prostate.

Ce prélèvement est ensuite analysé en laboratoire pour établir le diagnostic médical.

On note ici que les gestes exécutés lors d'une séance de curiethérapie sont très semblables techniquement aux gestes exécutés lors d'une biopsie.

Dans le cas de la biopsie, on introduit une aiguille dans la prostate pour accomplir un prélèvement, tandis que, dans le cas de la curiethérapie, on introduit une pluralité d'aiguilles dans la prostate pour y déposer des grains radioactifs.

Dans les deux cas, les gestes sont effectués sous contrôle échographique.

Sur le plan de la cinématique, les deux gestes sont réalisés par un mouvement translatif de l'aiguille selon une dimension, ladite aiguille ne présentant qu'un degré de liberté avec la grille de repérage (voir notamment en figure 1c).

Le geste dépend de la fonctionnalité finale : il est sortant dans le cas de la biopsie (on prélève un échantillon de prostate), et entrant dans le cas de la curiethérapie (on dépose des grains radioactifs dans la prostate).

Différentes approches sont aujourd'hui proposées dans l'état de la technique pour robotiser les équipements porte-aiguille afin de commander de façon fiable le déplacement de l'aiguille ainsi que son insertion dans un organe tel que la prostate.

On connaît notamment le document WO 2011/023866 qui propose un système robotisé de contrôle pour le guidage d'une aiguille percutanée.

Dans ce document, on s'intéresse plus particulièrement à la gestion en temps réel des paramètres d'insertion de l'aiguille lors des bougés de la prostate.

La prostate est un organe déformable et mobile.

La prostate a donc tendance à se déformer et se déplacer à l'intérieur du corps.

Ceci est particulièrement vrai lorsqu'une aiguille de ponction ou de traitement exerce une pression sur la prostate.

On constate de la même façon que les ponctions réalisées lors des biopsies peuvent provoquer des lésions induisant un gonflement de la prostate au cours de l'intervention.

On constate enfin des déformations et des déplacements de la prostate lorsque le patient bouge ou même lorsqu'il respire.

Le document WO 2011/023866 gère donc ces bougés et cherche principalement à adapter le guidage de l'aiguille en fonction de ces bougés pour améliorer la précision des prélèvements ou des traitements localisés de la prostate.

Le Demandeur observe toutefois qu'une telle approche, bien qu'intéressante pour perfectionner la précision des systèmes existants, ne limite pas le nombre d'insertions d'aiguilles.

En utilisant la technologie proposée dans ce document, la durée de l'intervention n'est pas réduite de façon significative.

Ce document ne traite pas non plus la problématique liée aux œdèmes.

Une autre approche tend à reproduire le geste de la curiethérapie tel qu'il est fait par un radiothérapeute ou un urologue.

Selon cette approche, la grille, utilisée pour le repérage lors de l'insertion des aiguilles, est simulée par un système robotisé.

Ces systèmes de guidage se substituent à la grille en reproduisant ses trois degrés de liberté : deux degrés de liberté en translation axiale pour positionner le guide et un troisième degré de liberté en translation longitudinale pour permettre au praticien d'introduire l'aiguille.

De tels systèmes sont proposés notamment dans la publication intitulée *"*Dynamic intraoperative prostate brachytherapy using 3D TRUS guidance with robot assistance"par Z. Wei *et al.*

Dans une autre publication intitulée *"*Multi-channel robotic system for prostate brachytherapy" par Podder *et al,* il est divulgué un système d'insertion multi-aiguilles, permettant d'insérer plusieurs aiguilles en même temps dans la prostate.

Les bénéfices présentés par les auteurs de cette publication sont les suivants : une diminution du temps d'intervention, une réduction de l'œdème, et une réduction du nombre de calculs de recalage dans le couplage images-robot.

Une telle solution a pour avantage, en plus de la précision, de maintenir des gestes répétitifs.

Une approche alternative consiste à s'affranchir totalement de la grille de repérage, et à la remplacer par un système directionnel.

Certains systèmes robotisés ont ainsi été développés pour donner n'importe quelle incidence à l'aiguille lors de l'insertion.

Par exemple, dans la publication *"*Robotic assistance for ultrasound-guided prostate brachytherapy" par G. Fichtinger *et al,* il est proposé un système pouvant faire varier à la fois :
- la position du guide de l'aiguille de manière à reproduire virtuellement la grille, et
- l'orientation de ce guide.

Les auteurs de cette publication proposent ainsi un système robotisé de curiethérapie de la prostate avec des résultats sur des fantômes à la première phase des essais cliniques relativement intéressants.

Leur système est composé d'une sonde échographique 2D transrectal et d'un robot parallèle à la sonde échographique.

Le robot est configuré pour fixer et guider une aiguille de curiethérapie.

L'insertion de l'aiguille et l'injection des doses se font cependant manuellement.

Le Demandeur soumet cependant qu'aucune des solutions proposées jusqu'à présent n'est pleinement satisfaisante.

Dans la plupart des systèmes, les principaux problèmes rencontrés demeurent :
- la nécessité de charger les grains radioactifs pendant le geste sous contrôle échographique ;
- la précision dans le dépôt des vecteurs radio-opaques sur les cibles millimétriques ;
- la calibration du robot opérateur par rapport à la sonde d'imagerie ; et
- la gestion des doses.

On connaît également du document US 2003/0018232 A1 un système automatisé d'implantation pour l'implantation de grains à faible dose radio-isotope dans un patient au cours d'une procédure de brachythérapie de la prostate dans lequel un système automatisé de contrôle du déplacement selon l'axe Z et un système automatisé de contrôle du déplacement selon les axes X-Y contrôlent un système d'aiguille.

Un tel système présente l'inconvénient majeur de nécessiter de pénétrer le patient en autant de points que le nombre de cibles à atteindre dans la prostate, ce qui augmente fortement le risque de création d'œdèmes externes et donc les douleurs, voire les complications postopératoires pour le patient.

### Objet et résumé de la présente invention

La présente invention vise à améliorer la situation décrite ci-dessus.

La présente invention vise plus particulièrement à remédier aux différents inconvénients mentionnés ci-dessus en proposant un système de guidage autonome d'un équipement porte-aiguille pour un traitement interne d'au moins une tumeur localisée sur un organe tel que par exemple la prostate. Ce système de guidage autonome d'un porte-aiguille est défini dans la revendication 1. Le procédé de calibration selon l'invention est défini dans la revendication 19.

Le système selon l'invention permet au moins une insertion d'une aiguille dans l'organe.

Il peut s'agir par exemple d'une curiethérapie.

Le module informatique de traitement comporte en outre un circuit de traitement d'images configuré :
a) d'une part pour déterminer dans le modèle un contourage de l'organe et une grille de cibles virtuelles, et
b) d'autre part pour déterminer dans le modèle des cibles de traitement à atteindre et éventuellement des zones d'exclusion sur lesquelles le traitement est interdit.

Ces informations permettent de générer un planning de traitement comprenant les coordonnées des cibles de traitement (et éventuellement celles des zones d'exclusion d'organes à risque) dans le référentiel de la sonde d'imagerie.

De préférence, le planning de traitement est enregistré (localement ou de façon déportée) sur des moyens électroniques de mémorisation.

Le module informatique de traitement comporte également un calculateur qui est configuré pour calculer à partir du planning les coordonnées, dans le référentiel du robot de guidage, des cibles de traitement et éventuellement celles des zones d'exclusion.

Préférentiellement, le calculateur est apte à transformer les coordonnées d'un point dans le référentiel de la sonde d'imagerie pour les convertir dans le référentiel du robot de guidage.

Ce calcul permet de générer les instructions de guidage pour guider avec précision la tête de l'aiguille de manière à atteindre chacune des cibles de traitement.

Ainsi, grâce à cet agencement de moyens techniques, caractéristique de la présente invention, on dispose d'un système robotisé, fiable et autonome, capable de guider l'aiguille dans ses déplacements pour atteindre les cibles de traitement et éviter les zones dangereuses (par exemple l'urètre dans laquelle il ne faut pas déposer de grains radioactifs).

Le système selon l'invention peut ainsi réaliser une insertion guidée sur n'importe quels tissus mous avec une précision millimétrique.

Un tel système peut être utilisé aussi bien pour les interventions du type curiethérapie que pour des biopsies.

Le système selon la présente invention permet une robotisation autonome pour atteindre rapidement et avec une grande précision les cibles tumorales, ce qui équivaut à une robotisation complète du geste de la curiethérapie et/ou de la biopsie.

Le robot comprend un bras articulé qui permet le guidage de l'aiguille en fonction des instructions de guidage reçues du module informatique de traitement. Ces instructions de guidage comprennent des informations relatives aux déplacements ainsi qu'à l'inclinaison et l'orientation de l'aiguille par rapport à l'organe et à la cible de traitement. A cet effet le bras articulé peut comporter au moins six-axes de liberté de manière à permettre le déplacement de l'aiguille dans les trois directions de l'espace et le changement de l'inclinaison et l'orientation de l'aiguille grâce aux trois rotations autour de ces trois directions. Le robot de guidage multidirectionnel peut être redondant, à 7 degrés de liberté pour avoir plusieurs postures dans un environnement contraignant, permettant d'atteindre les cibles de traitement ;

Avantageusement, le calculateur est configuré pour générer les instructions de guidage de manière à ce que, pour atteindre chacune des cibles de traitement, l'aiguille traverse la peau du patient selon un unique point d'accès.

Ceci rend beaucoup moins douloureux l'intervention par rapport à la pratique conventionnelle où plusieurs aiguilles doivent pénétrer le patient.

Grâce à l'invention, le nombre d'œdèmes diminue de façon très significative.

Avantageusement, le calculateur peut être configuré pour déterminer les instructions de guidage de manière à ce que, entre deux cibles de traitement, l'aiguille est retirée de l'organe sans être retirée du corps.

Bien évidemment, on peut toutefois prévoir plusieurs points d'accès ; ceci est vrai par exemple pour atteindre toutes les cibles de traitement qui ont été déterminées. Il est en effet possible que l'insertion par un unique point d'accès ne permette pas d'atteindre toutes les cibles de traitement.

Une telle approche est possible grâce à la robotisation du guidage et à la cavité entourant la prostate. Il existe en effet un espace dépourvu d'organes à risque ; cet espace représente quelques centimètres entre la couche épidermique et la prostate.

C'est cet espace qui est ici exploité avec cette nouvelle technique pour atteindre chacune des cibles sans faire ressortir l'aiguille du corps du patient ; les instructions de guidage sont en effet générées par le calculateur de telle façon à ce que l'aiguille reste dans cette cavité après avoir atteint une première cible et change d'inclinaison pour atteindre la deuxième cible.

Plus particulièrement, le calculateur calcule les coordonnées du déplacement et de l'orientation de l'aiguille pour passer d'une première à une deuxième cible sans ressortir complètement du corps du patient.

Avantageusement, le module informatique de traitement comporte un circuit de dosimétrie ; ce circuit est configuré pour déterminer une dose de produit à injecter pour chaque cible de traitement en fonction d'une estimation du volume de ladite au moins une tumeur.

De préférence, l'estimation du volume de ladite au moins une tumeur est obtenue par une analyse de l'image de la prostate (reconnaissance de forme, etc.).

Il est ainsi possible de gérer automatiquement la distribution des différentes doses à injecter.

Avantageusement, le circuit de dosimétrie est configuré pour mettre à jour le planning de traitement de manière adaptative de sorte qu'une dose soit associée à chacune des cibles de traitement : la dose à déposer sur chaque cible de traitement peut être calculée automatiquement, suivant la dimension de la cible, l'inclinaison et l'orientation de l'aiguille et l'emplacement des autres cibles avoisinants déjà traités.

On peut par exemple prévoir que le circuit de dosimétrie transmet aux moyens de mémorisation un signal numérique comprenant une information relative à chacune des doses.

On connaît ainsi dans le planning de traitement la dose à injecter pour chacune des cibles définies.

Avantageusement, l'équipement porte-aiguille selon la présente invention comporte un chargeur connecté à la base de l'aiguille.

De préférence, le chargeur comprend en son intérieur un produit destiné à être injecté dans au moins une des cibles de traitement.

Par exemple, ce produit consiste en des grains radioactifs du type I125.

Avantageusement, ce chargeur est connecté directement ou indirectement au circuit de dosimétrie de manière à recevoir l'information relative à la dose à administrer pour la cible visée.

Dans un mode de réalisation avantageux, l'équipement porte-aiguille comporte un mandrin mobile apte à coulisser à l'intérieur de l'aiguille ; ce mandrin est de préférence configuré pour passer à travers le chargeur de manière à acheminer mécaniquement le produit à injecter à travers l'aiguille jusqu'à la tête de l'aiguille.

On peut donc considérer que le mandrin et l'aiguille coopèrent ensemble pour fonctionner comme une seringue permettant l'injection du produit dans chacune des cibles de la prostate.

Avantageusement, l'équipement porte-aiguille comporte des moyens de motorisation configurés pour motoriser le déplacement linéaire du mandrin à l'intérieur de l'aiguille.

De préférence, les moyens de motorisation du mandrin sont synchronisés avec le robot de guidage en fonction des instructions de guidage du robot et de la dosimétrie définie dans le planning de traitement.

Cette synchronisation du mandrin et du robot permet de reproduire avec une grande précision le geste du chirurgien.

On comprendra ici que cette synchronisation est programmée : elle dépend notamment du type d'intervention (curiethérapie ou biopsie), de la dose et de la cible.

Plus particulièrement, la cinématique du système est une combinaison de deux mouvements qui sont :
- le déplacement linéaire du robot qui permet l'insertion et le retrait de l'aiguille dans la prostate, et
- le déplacement du mandrin par les moyens de motorisation dans l'aiguille pour déposer linéairement les grains dans la prostate.

Dans un mode de réalisation particulier, le produit à injecter comporte une pluralité de grains radioactifs, par exemple du type I125.

Dans ce mode, il est prévu de préférence que l'équipement porte-aiguille comporte un capteur de radiations, positionné à la sortie du chargeur, pour détecter le passage d'un grain radioactif du chargeur vers l'aiguille.

La présence d'un tel capteur est souhaitable pour contrôler la dose radioactive injectée dans le corps du patient. Elle permet de sécuriser le système pour éviter notamment une surexposition du patient.

Avantageusement, le chargeur comporte un actionneur pas-à-pas configuré pour charger l'aiguille d'un grain radioactif.

Selon un mode de réalisation, le calculateur est configuré pour générer lesdites instructions de guidage de manière à ce que, pour atteindre chacune des cibles de traitement, le robot opère une rotation de l'aiguille autour de son axe longitudinal lors de l'insertion de l'aiguille dans l'organe : une telle rotation lors de l'insertion présente pour intérêt de minimiser l'effet des frottements statiques sur l'axe longitudinal de l'aiguille.

Dans un mode de réalisation avantageux, la base de l'aiguille est instrumentée d'un capteur de force à six axes.

De préférence, ce capteur est configuré pour détecter les différentes contraintes exercées sur l'aiguille (par exemple, les contraintes exercées sur l'aiguille lors de l'insertion de l'aiguille dans l'organe).

Avantageusement, le calculateur est apte à corriger en temps réel les instructions de guidage pour modifier l'orientation de l'aiguille dans l'organe ; une telle correction est réalisée par le calculateur lorsque par exemple la somme des contraintes mesurées par le capteur de force est supérieure à un seuil déterminé.

De préférence, le calculateur est configuré pour modifier l'orientation de l'aiguille de manière à ce que la somme des contraintes mesurées par le capteur de force soit sensiblement égale à zéro.

Dans un mode de réalisation avantageux, le module informatique de traitement comporte des moyens de détection du bougé de l'organe.

De préférence, ces moyens comprennent :
- un réseau interne de géolocalisation comprenant au moins une première balise ancrée sur l'organe (qui peut être mobile comme la prostate) et au moins trois deuxièmes balises ancrées sur des tissus fixes dans le corps du patient.
- un circuit de détection configuré pour déterminer la position de chacune des balises ;
- des moyens de calcul configurés pour calculer en fonction de la position de chacune des balises les variations de la distance entre la première balise et chacune des deuxièmes balises afin de détecter le bougé de l'organe, lors par exemple d'une insertion d'une aiguille dans l'organe.

De préférence, on sélectionne des balises du type puce RFID.

Calibrer le système est caractéristique de la présente invention.

Dans un mode de réalisation avantageux, le système selon la présente invention comporte un support, destiné à recevoir le patient, sur lequel est fixée la sonde d'imagerie : ce support de la sonde d'imagerie est de préférence un élément indépendant et détaché du robot de guidage. Cela permet de pré-positionner respectivement le robot de guidage multidirectionnel et le support de la sonde d'imagerie indépendamment l'un de l'autre par rapport au corps du patient.

Dans ce mode, le module informatique de traitement comporte un module de calibration présentant une caméra fixée sur le robot de guidage et une étiquette positionnée sur le support.

De préférence, la caméra est configurée pour détecter l'étiquette sur le support afin de déterminer, dans le référentiel du robot, la position relative de la sonde d'imagerie par rapport au robot de guidage.

Il est également souhaitable que le module de calibration comprenne un laser apte à émettre un faisceau laser formant un plan.

Le module de calibration est alors configuré pour déterminer la position de la tête de l'aiguille en faisant déplacer l'aiguille dans le plan formé par le laser et en détectant avec la caméra la position de la tête de l'aiguille.

Corrélativement, un des autres aspects de la présente invention est de proposer un procédé de calibration pour calibrer le système décrit ci-dessus.

La calibration du système selon l'invention comporte :
- une première phase au cours de laquelle le module de calibration détermine la position et l'orientation de l'aiguille dans le référentiel du robot de guidage, et
- une deuxième phase au cours de laquelle le module de calibration détermine la position et l'orientation de la sonde d'imagerie dans le référentiel du robot de guidage.

Dans un mode de réalisation avantageux, la première phase comporte les étapes suivantes :
- une étape d'éclairage au cours de laquelle une source lumineuse du type laser en ligne émet en direction d'un panneau fixe ;
- une étape de déplacement au cours de laquelle l'aiguille est déplacée par le robot dans le champ entre la source et le panneau selon un mouvement translatif de manière à traverser le plan formé par ladite source ;
- une étape de détermination au cours de laquelle on détermine la position et l'orientation de l'aiguille dans le référentiel du robot de guidage en détectant sur le panneau, à l'aide d'une deuxième caméra numérique, l'intersection de l'aiguille avec le plan formé par la source.

Dans un mode de réalisation avantageux dans lequel le système comporte un support destiné à recevoir le patient sur lequel est fixée la sonde d'imagerie, on peut prévoir que la deuxième phase du procédé de calibration comporte une étape de détection au cours de laquelle une première caméra numérique détecte une étiquette positionnée sur le support afin de déterminer, dans le référentiel du robot, la position relative de la sonde d'imagerie par rapport au robot de guidage.

Ainsi, grâce à ces différentes caractéristiques techniques, structurelles et fonctionnelles, la présente invention propose un système mécatronique automatisé et autonome, composé d'un ensemble d'actionneurs électriques et de capteurs intelligents, faisant abstraction de la grille physique d'insertion et de guidage ; un tel système permet notamment :
- de réduire les œdèmes en limitant le nombre d'insertions d'aiguille dans le corps du patient (utilisation d'une seule aiguille contrairement à une vingtaine d'aiguilles utilisées dans l'approche classique) pour diminuer les effets secondaires (incontinence et impuissance) et le traumatisme pour le patient,
- d'améliorer la fiabilité et la précision des dépôts des grains radioactifs tout en accélérant la prise en charge, et
- d'automatiser la charge de l'aiguille avec des grains radioactifs pour réduire les manipulations de la source radioactive par le praticien.

### Brève description des figures annexées

D'autres caractéristiques et avantages de la présente invention ressortiront de la description ci-dessous, en référence aux figures annexées qui en illustrent un mode de réalisation dépourvu de tout caractère limitatif et sur lesquelles :
- les figures 2a et 2b représentent chacune de façon schématique une vue en perspective d'un système de guidage autonome d'un équipement porte-aiguille selon un exemple de réalisation ;
- la figure 3 représente de façon schématique une vue en perspective d'un équipement porte-aiguille avec un mandrin ;
- les figures 4a et 4b représentent de façon schématique l'insertion d'une aiguille dans le corps d'un patient avec un système de guidage conforme à l'exemple des figures 2a et 2b ;
- la figure 5 représente de façon schématique le positionnement d'un réseau interne de géolocalisation dans le corps du patient pour gérer en temps réel les bougés de la prostate du patient pendant une intervention ;
- la figures 6a et 6b représentent respectivement un graphique illustrant, pour un test réalisé, la position de la tête de l'aiguille par rapport à la cible à atteindre, ainsi que les erreurs de positionnement de l'aiguille par rapport à la cible ;
- les figures 7a, 7b et 7c représentent les images de la prostate issues des différentes phases de traitement préopératoire pour calculer les instructions de guidage dans la prostate du patient ;
- la figure 8 représente de façon schématique le module de calibration utilisé pour déterminer la position de la tête de l'aiguille dans le référentiel du robot de guidage ;
- la figure 9 représentant un organigramme des différentes étapes mises en œuvre informatiquement par le module de traitement du robot ; et
- la figure 10 représente de façon schématique le module informatique de traitement selon un exemple de réalisation utilisable dans le système de la présente invention.

### Description détaillée d'un exemple de réalisation de l'invention

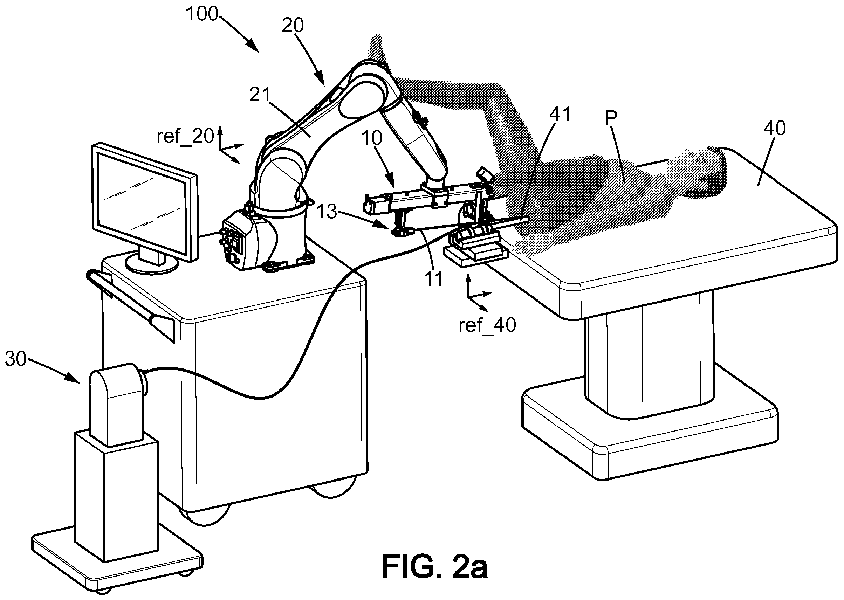

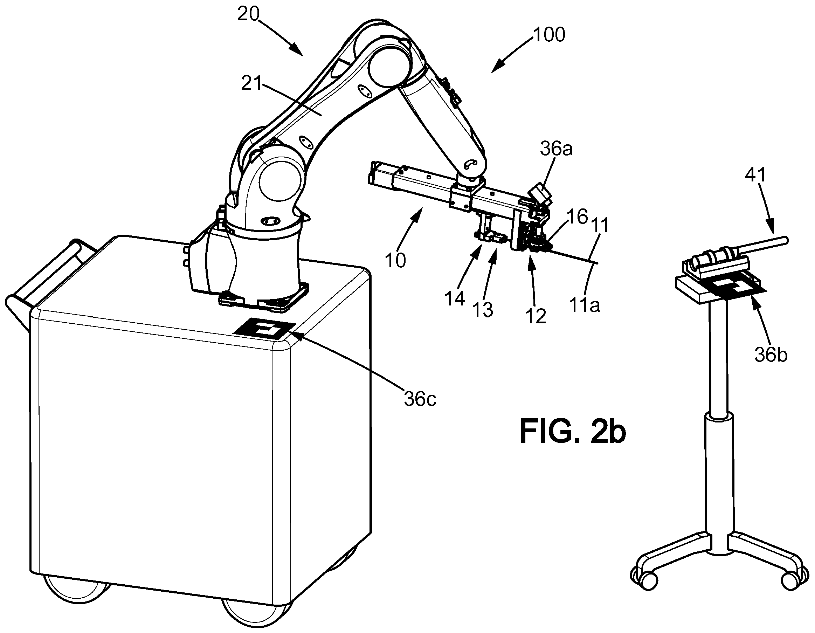

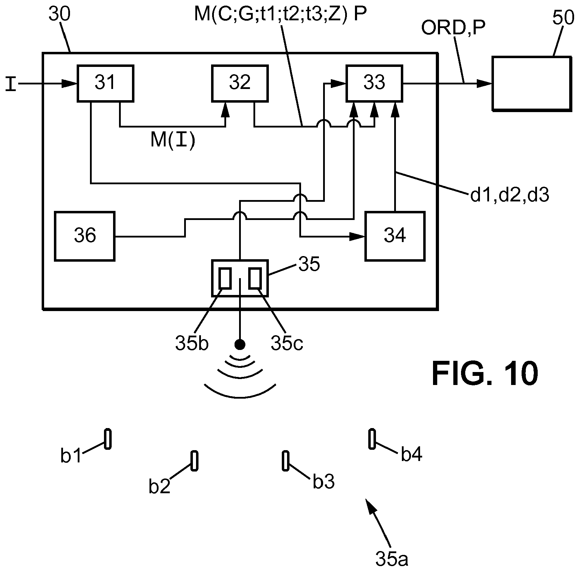

Un système de guidage autonome d'un équipement porte-aiguille selon un exemple de réalisation de la présente invention va maintenant être décrit dans ce qui va suivre en faisant référence conjointement aux figures 2 à 10.

La description qui va suivre porte essentiellement sur un exemple pour le traitement radio-thérapeutique (du type curiethérapie) de la prostate.

L'homme du métier comprendra ici qu'il s'agit d'un exemple parmi d'autres, le système décrit pouvant être utilisé sur d'autres organes que la prostate et pour d'autres applications (la biopsie par exemple).

Ainsi, l'homme du métier comprendra notamment que le système proposé dans le cadre de la présente invention peut s'adapter par exemple à la curiethérapie utérovaginale ou la curiethérapie endo-luminale comme la curiethérapie endo-bronchique ou endo-oesophagienne, le but étant ici d'implanter des sources radioactives au contact d'un tissu à irradier, en profitant des cavités naturelles qui enveloppent les grains radioactifs.

Dans l'exemple décrit ici, on dispose donc d'un système robotisé 100 qui permet le traitement radio-thérapeutique de tumeurs localisées au niveau de la prostate d'un patient.

Le système 100 décrit dans cet exemple permet de pratiquer sur la prostate d'un patient la technique de curiethérapie de façon automatisée et intelligente à travers un guidage sous imagerie médicale.

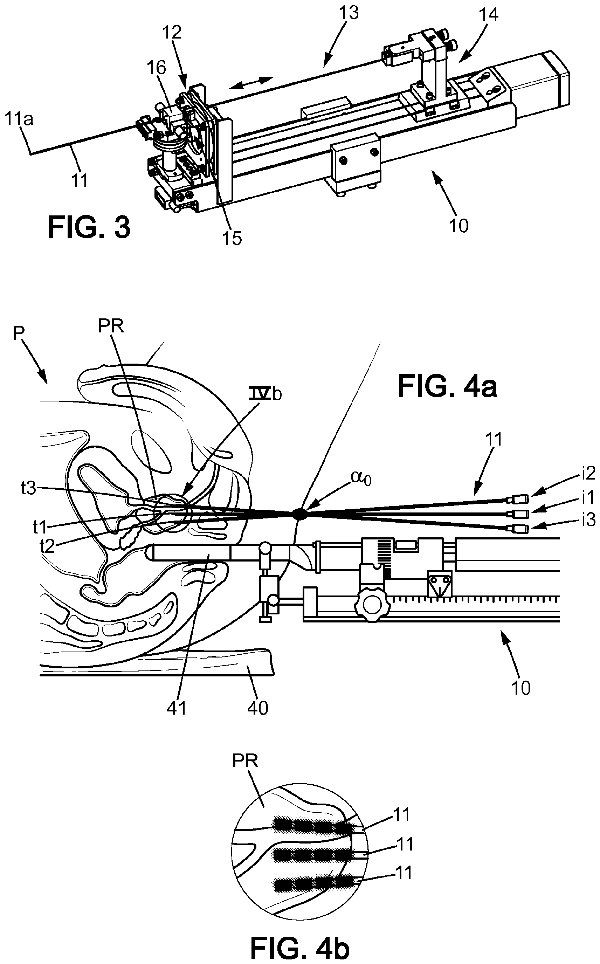

Plus particulièrement, le système 100 décrit dans cet exemple permet d'automatiser de façon intégrée les opérations suivantes :
- insertion d'une aiguille 11 à travers un unique point d'accès du corps du patient, noté ici α0 (voir en figure 4) ;
- chargement automatique de l'aiguille 11 ;
- injection de grains radioactifs à faible et à haut débits ;
- extraction de tissus tumoraux (pour le cas spécifique de la biopsie) ;
- calibration de l'aiguille 11.

Plus particulièrement, le système 100 décrit ici comprend classiquement un support 40 destiné à recevoir le patient P en position allongée (comme illustré en figure 2a).

Une sonde d'imagerie 41 telle qu'une sonde échographique est fixée solidairement sur ce support 40.

Dans cet exemple, le système 100 comprend en outre un équipement porte-aiguille 10. Cet équipement 10 sert de préhenseur pour l'aiguille 11.

Dans cet exemple, l'équipement 10 est monté sur un robot de guidage multidirectionnel 20.

On remarque que le robot de guidage multidirectionnel 20, d'une part, et le support 40 de la sonde d'imagerie 41, d'autre part, sont physiquement indépendants, par exemple montés respectivement sur deux chariots distincts. Le robot de guidage multidirectionnel 20 et le support 40 peuvent être avantageusement pré-positionnés indépendamment l'un de l'autre par rapport au patient, avant la mise en œuvre d'une séance : une calibration préalable du système est de préférence exécuter pour connaître les coordonnées de la sonde d'imagerie 41 par rapport au robot de guidage 20 dans le référentiel du robot (noté sur la figure 2a : ref_20).

Ce robot 20 comprend un bras articulé 21 qui permet le guidage de l'aiguille 11 en fonction d'instructions de guidage ORD reçues du module informatique de traitement 30. Comme illustré, ce bras robotisé est un robot six-axes, offrant six axes de liberté : le bras robotisé permet le déplacement de l'aiguille dans les trois directions de l'espace (Ox ; Oy ; Oz) et le changement de l'inclinaison et l'orientation de l'aiguille grâce aux trois rotations autour de ces trois directions (autour de Ox ; autour de Oy ; autour de Oz). Le bras robotisé peut encore être redondant, à 7 axes (7 axes de liberté), afin d'avoir une redondance sur la posture du robot suivant la position du patient.

Ces instructions de guidage ORD comprennent des informations relatives aux déplacements ainsi qu'à l'inclinaison et l'orientation de l'aiguille 11 par rapport à la prostate PR du patient P.

La génération de ces instructions de guidage ORD est caractéristique de la présente invention.

Elle nécessite en phase préopératoire une succession d'étapes techniques spécifiques par le module de traitement 30.

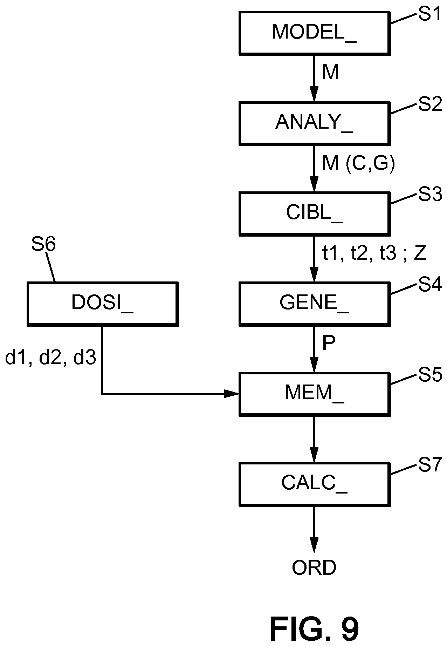

Ces étapes représentées en figure 9 sont décrites ci-dessous.

Dans l'exemple décrit, il est ainsi prévu une sonde d'imagerie 41 telle qu'une sonde échographique qui fournit au moins une image I de la prostate PR du patient P.

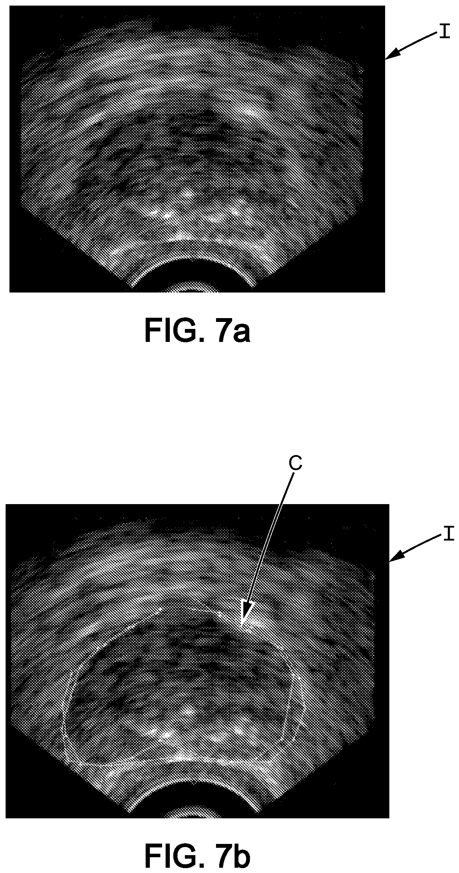

Comme illustré en figure 7a, l'image I représentant la prostate est traitée par un circuit de modélisation 31 qui génère lors d'une étape S1 un modèle numérique M de la prostate PR.

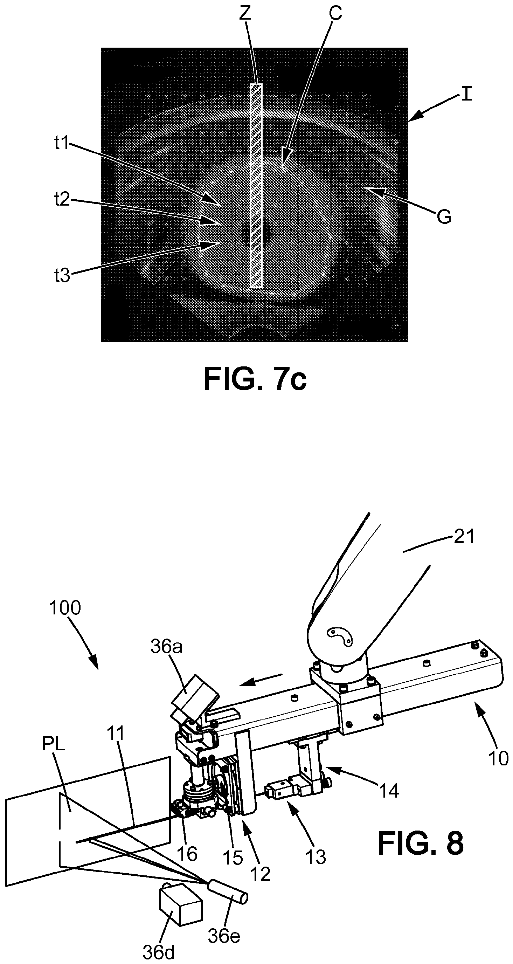

Ce modèle M est ensuite analysé lors d'une étape S2 par un circuit de traitement d'images 32. Au cours de cette analyse, on détermine un contourage C de la prostate et on génère dans le modèle M une grille de cibles G (voir en figure 7b et 7c).

Ce contourage C peut par exemple être réalisé à l'aide d'un algorithme de segmentation.

La grille G est une grille virtuelle remplaçant la grille de repérage utilisée dans les approches classiques (illustrées en figures la et 1c).

Cette grille virtuelle G est superposée à la prostate PR dans le modèle M.

Lors d'une étape S3, le circuit 32 détermine (voir en figure 7c) dans le modèle M des cibles de traitement à atteindre t1, t2 et t3, et des zones d'exclusion Z sur lesquelles le traitement est interdit (c'est-à-dire des zones dans lesquelles l'aiguille 11 ne peut entrer : par exemple l'urètre pour le cas spécifique de la prostate).

Les cibles t1, t2 et t3 admissibles au traitement sont ensuite englobées dans le contourage C.

A partir de ces informations fournies par le modèle numérique M, le circuit 32 génère ensuite un planning de traitement P lors d'une étape S4.

Ce planning P est caractéristique de la présente invention.

Le planning de traitement comprend les coordonnées cartésiennes de chacune des cibles de traitement t1, t2 et t3 et des zones d'exclusion Z dans le référentiel de la sonde d'imagerie 41.

Ce planning P est enregistré localement ou de façon déportée sur des moyens électroniques de mémorisation 50, ceci lors d'une étape S5.

Dans cet exemple, le module 30 comprend en outre un calculateur 33 qui calcule lors d'une étape S7 les coordonnées des cibles de traitement t1, t2 et t3 et des zones d'exclusion Z dans le référentiel du robot de guidage.

Cette transformation des coordonnées des cibles de traitement t1, t2 et t3 et des zones d'exclusion Z dans le référentiel du robot de guidage 20 permet de générer les instructions de guidage ORD du robot de guidage 20 pour guider la tête de l'aiguille 11 et atteindre chacune des cibles de traitement t1, t2 et t3, sans entrer dans une zone Z prohibée de la prostate.

Pour faire cette transformation, il est souhaitable de connaître les coordonnées de la sonde d'imagerie 41 par rapport au robot de guidage 20 dans le référentiel du robot (noté sur la figure 2a : ref_20).

Pour ce faire, il est souhaitable de calibrer au préalable le système.

Une des problématiques soulevées par la calibration du système est ici de faire le lien entre des positions identifiées sur une image échographique I de la prostate PR et ces mêmes positions dans le repère lié au robot 20, le but étant au final d'être capable de déterminer la position et l'orientation de la sonde échographique 41 dans le repère du robot pour guider avec précision l'aiguille 11 dans le corps du patient (et notamment sa prostate).

Lorsque l'on atteint une cible t1, t2 ou t3 dans la prostate PR, la tête de l'aiguille 11 et la cible en question sont confondues.

Il faut déterminer la transformation permettant de passer du repère de la sonde (noté dans la figure 2a : ref_40) à celui du robot. Cette transformation dépend du positionnement du robot 20 et de la sonde échographique 41.

Dans l'exemple décrit ici, et comme illustré en figures 2b et 8, le système 100 comprend un module de calibration 36 avec une caméra 36a capable d'identifier des marqueurs dont :
- un premier marqueur, noté 36c, se présentant sous la forme d'une étiquette et dont la position sur le robot 20 est connue ; ce premier marqueur 36c positionné sur le robot 20 permet ainsi de calibrer la caméra 36a (dite première caméra) par rapport au robot 20, et
- un deuxième marqueur, noté 36b, se présentant sous la forme d'une étiquette positionnée sur le support 40.

Cette étiquette 36b est utilisée pour estimer la position de la sonde échographique 41 par rapport à la première caméra 36a, puis par rapport à celle du robot.

Une des autres problématiques soulevées par la calibration du système 100 est de connaître précisément la position et l'orientation de l'aiguille 11 dans le repère du robot.

Il faut donc centrer le système et déterminer les coordonnées de la tête 11a de l'aiguille 11. Cette tête 11a correspond au centre du système 100 ; c'est cette tête 11a qui doit atteindre chacune des cibles t1, t2 et t3.

Ceci se fait par un scan 3D de l'aiguille 11.

Comme illustré en figure 8, on prévoit que le module de calibration 36 comporte en outre une caméra 36d (dite deuxième caméra) calibrée et un laser 36e de type ligne.

Le rayon de ce laser 36e forme un plan PL dans l'espace, ce plan étant connu dans le repère de la caméra.

Lorsqu'un objet coupe le plan PL du laser 36e, cette intersection est visible dans l'image de la caméra 36d ; il est alors possible de déterminer la position exacte de l'intersection dans le repère caméra.

En utilisant ce principe, et en supposant que l'aiguille 11 coupe le plan PL du laser de manière ponctuelle, on peut en déplaçant l'aiguille 11 avec le robot 20 devant le laser 36e calculer plusieurs positions 3D le long de l'aiguille.

Ce nuage de points permet ensuite de reconstruire numériquement l'aiguille 11 et d'en déduire le centre d'outil (c'est-à-dire la position de la pointe de l'aiguille 11) et son orientation.

Il est préférable de réaliser cette opération de centrage avant chaque intervention.

Le planning de traitement P comporte en outre les doses à injecter pour chaque cible.

Dans cet exemple, le module informatique de traitement 30 comporte un circuit de dosimétrie 34 qui permet, lors d'une étape S6, de déterminer les doses d1, d2 et d3 de grains radioactif à injecter respectivement pour chacune des cibles de traitement t1, t2 et t3.

Dans l'exemple décrit ici, les doses d1, d2 et d3 sont déterminées en fonction d'une estimation du volume de la tumeur à traiter.

Dans cet exemple, cette estimation est obtenue par un traitement d'image consistant à évaluer ce volume en fonction d'une estimation des dimensions de la prostate et de la tumeur à traiter.

Le système selon la présente invention permet un chargement automatique de l'aiguille 11 en fonction des doses d1, d2 et d3 du planning P pour limiter les manipulations du praticien et utiliser une unique aiguille 11 pendant toute la durée de l'intervention.

Dans l'exemple décrit ici et comme illustré en figure 3, l'équipement porte-aiguille 10 comporte donc un chargeur 12.

Dans cet exemple, le chargeur 12 est connecté directement ou indirectement à la base de l'aiguille 11.

Dans cet exemple, le chargeur 12 comprend des grains radioactifs du type I125.

On comprendra ici que ces grains sont destinés à être injectés dans les cibles de traitement t1, t2 et t3.

Pour le chargement de ces grains dans l'aiguille 11, il est prévu un actionneur pas à pas (non illustré ici) qui permet de positionner un par un les grains dans l'aiguille.

Il est souhaitable de s'assurer que la distribution des grains dans l'aiguille 11 se déroule correctement.

Ainsi, dans l'exemple décrit ici, il est prévu un capteur de radiations 15, positionné à la sortie du chargeur 12, pour détecter le passage d'un grain radioactif du chargeur 12 vers l'aiguille 11.

Un tel capteur 15 assure la sécurité du système d'une part pour éviter une trop forte exposition de l'organe en éléments radioactifs, et d'autre part pour éviter qu'aucun grain ne soit introduit dans l'organe.

Pour l'acheminement du grain dans l'organe, l'équipement porte-aiguille 10 comporte un mandrin 13 coopérant avec l'aiguille 11 pour fonctionner comme une seringue.

Plus particulièrement, le mandrin 13 peut coulisser à l'intérieur de l'aiguille 11 pour passer à travers le chargeur 12 de manière à acheminer mécaniquement le grain à injecter à travers l'aiguille 11 jusqu'à la tête de celle-ci.

De préférence, il est prévu que le mandrin 13 soit motorisé par des moyens de motorisation 14 synchronisés avec le robot de guidage 20 pour reproduire le geste du chirurgien lors d'une curiethérapie classique.

Le traitement réalisé en utilisant le système selon l'invention comprend l'insertion d'une seule aiguille 11 dans le corps du patient. Cette aiguille va ensuite injecter un produit sur les différentes cibles de traitement t1, t2 et t3 suivant le planning de traitement P.

En effet, il est prévu d'effectuer avec le même point d'accès au corps α₀ plusieurs insertions i1, i2 et i3 dans la prostate pour atteindre respectivement les cibles t1, t2 et t3.

Le planning P comprend donc les coordonnées de chaque cible prostatique t1, t2 et t3 en position et en orientation.

A titre d'exemple et comme illustré en figure 4a, la première insertion, notée i1, à travers le point d'accès α₀ pour atteindre la cible t1 se fait dans une direction sensiblement horizontale.

Les coordonnées de la cible t1 sont déterminées à partir de la grille virtuelle G.

Le planning de traitement P permet de calculer la dose d1 de radiation associée à cette cible t1. L'aiguille 11 est ainsi chargée à partir du chargeur 12 de grains, en insérant un grain à la fois dans l'aiguille 11 à l'aide du mandrin 13.

L'insertion il se fait sous guidage échographique, jusqu'à la cible t1.

Comme illustré en figure 4b, le dépôt des grains sur chaque cible se fait de façon rectiligne, grâce à deux mouvements synchronisés : la pointe de l'aiguille 11, en utilisant le robot 20 vers l'extérieur de la prostate et le mandrin 13 en translation à l'intérieur de la prostate à partir du moteur linéaire. Lors de ce déplacement rectiligne, le robot peut avantageusement opérer une rotation de l'aiguille 11 autour de son axe longitudinal: une telle rotation lors de l'insertion présente pour intérêt de minimiser l'effet des frottements statiques sur l'axe longitudinal de l'aiguille.

Une fois le dépôt terminé, l'aiguille 11 est retirée de la prostate mais pas du corps.

Une deuxième cible t2 est choisie sur la grille virtuelle G avec un angle d'incidence différent de la première cible t1.

Le robot 20 est alors orienté pour effectuer une deuxième insertion i2 à partir de α0 en inclinant l'aiguille 11 selon l'angle d'inclinaison défini dans le planning P.

Ainsi, la deuxième cible t2 est alors atteinte, et le dépôt des grains se fait comme pour la deuxième cible t2.

Il est toutefois noté que les instructions de guidage ORD sont telles que l'emplacement des nouveaux grains est recalculé pour que les grains déposés lors de la deuxième insertion ne chevauchent avec les grains déposés lors de la première insertion.

On procède ensuite à une troisième insertion i3 pour atteindre la cible t3 en respectant les mêmes contraintes et en passant toujours à travers le même point d'accès α₀ sans ressortir du corps du patient.

Permettre un guidage adaptatif est également un des objectifs de la présente invention.

Il est ainsi prévu dans un exemple de réalisation particulier d'instrumenter la base de l'aiguille 11 avec un capteur de force 16 à six axes.

Dans cet exemple, le capteur 16 peut donc détecter les différentes contraintes exercées sur l'aiguille 11 lors par exemple de l'insertion de l'aiguille 11 dans l'organe PR.

Dans cet exemple, les informations relatives aux contraintes exercées sur l'aiguille 11 sont envoyées au calculateur 33 qui peut ensuite corriger en temps réel les instructions de guidage ORD pour modifier l'orientation de l'aiguille 11 dans l'organe si la somme des contraintes mesurées par ledit capteur de force 16 est supérieure à un seuil déterminé.

Plus particulièrement, il devient possible dans cet exemple de modifier l'orientation de l'aiguille 11 pour que la somme des contraintes mesurées par le capteur de force 16 soit sensiblement égale à zéro.

Il est également prévu dans cet exemple un guidage adaptif permettant de tenir compte des bougés de la prostate PR lors par exemple d'une insertion de l'aiguille 11 dans la prostate.

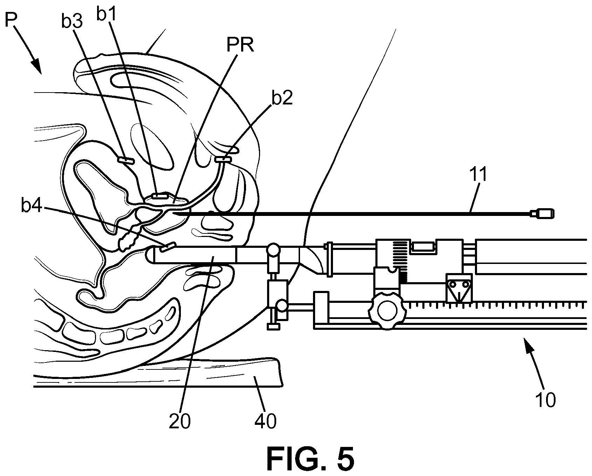

Pour gérer le guidage de l'aiguille 11 en cas de bougés de la prostate PR, il est prévu dans le cadre de la présente invention (voir en figure 5) des moyens de détection 35 spécifiques comprenant un réseau interne de géolocalisation 35a avec une pluralité de balises (par exemple des puces RFID) dont :
- une balise b1 ancrée sur une paroi de la prostate, et
- (au moins) trois autres balises b2, b3 et b4 ancrées sur des tissus fixes dans le corps du patient.

Ces moyens de détection 35 comprennent également un circuit de détection 35b pour déterminer la position de chacune des balises b1, b2, b3 et b4 et des moyens de calcul 35c capables de calculer les variations des distances d(b1, b2), d(b1, b3) et d(b1, b4) entre la balise b1 et chacune des autres balises b2, b3, b4 afin de détecter le bougé de l'organe.

Grâce à cet agencement de moyens techniques, il est possible de connaître en temps réel les bougés de la prostate et de fournir ces informations au calculateur 33 qui va recalculer les instructions de guidage ORD pour tenir compte des déplacements et des déformations de la prostate PR.

Le principe de la géolocalisation du mouvement de la prostate pendant le geste de curiethérapie se base donc sur le placement précis et localisé des puces RFID.

Avec un système extérieur d'émission et de réception de signaux RFID, les puces b1, b2, b3, b4 sont capables de réagir chacune suivant leur emplacement en répondant par des signaux sensibles en amplitude aux mouvements de la prostate.

Ces variations permettent suivant un calcul géométrique de déduire le déplacement relatif de la prostate par rapport à la localisation initiale de la prostate avant le début du traitement.

Le système 100 proposé dans le cadre de la présente invention peut effectuer des dépôts automatisés de grains radioactifs dans un espace tumoral localisé avec une précision millimétrique.

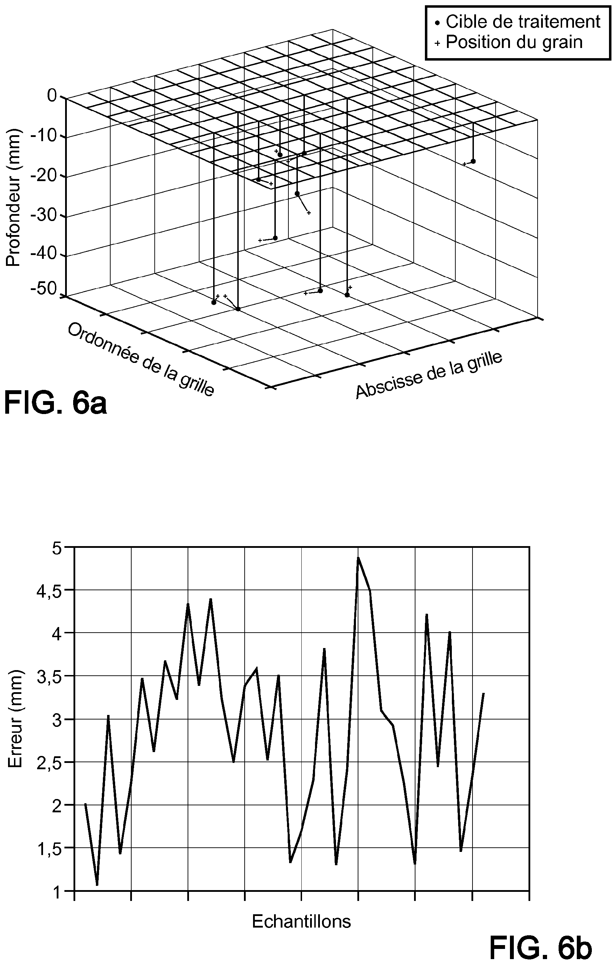

En effet, les résultats illustrés en figures 6a et 6b montrent que lors d'un test clinique réalisé sur des fantômes de prostate (sans guidage adaptatif) l'aiguille 11 atteint les différentes cibles avec une erreur de moins de 5 millimètres, ce qui est conforme aux normes de sécurité.

Ces résultats sont très encourageants, car ils démontrent les performances du système en termes de précision et de fiabilité.

Ainsi, la présente invention présente de nombreux avantages techniques parmi lesquels on peut citer notamment :
- la réduction du temps d'intervention (20 minutes pour une séance de curiethérapie contre 1 heure 30 minutes avec les techniques classiques, 5 minutes pour la biopsie) ;
- l'intégration sur un seul dispositif médical robotisé, à la fois les fonctionnalités de l'insertion de l'aiguille de curiethérapie, du chargement automatique des grains radioactifs et de l'injection avec un dépôt précis sous un guidage par imagerie médicale, et
- une diminution des œdèmes : les opérations de dépôts de grains se font à partir d'un seul accès de la peau. Ceci rend beaucoup moins douloureuse l'intervention par rapport à la pratique conventionnelle où plusieurs aiguilles doivent pénétrer le patient.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Système de guidage (100) autonome d'un équipement porte-aiguille (10) pour un traitement interne d'au moins une tumeur localisée sur un organe (PR), ledit traitement impliquant au moins une insertion d'une aiguille (11) dans ledit organe (PR),
dans lequel ledit équipement (10) est monté sur un robot de guidage multidirectionnel (20) apte à recevoir des instructions de guidage (ORD),
dans lequel ledit système (100) comporte un module informatique de traitement (30) comprenant :
- un circuit de modélisation (31) configuré pour générer un modèle numérique (M) de l'organe à partir d'au moins une image (I) dudit organe (PR) fournie par une sonde d'imagerie (41),
- un circuit de traitement d'images (32) configuré :
a) d'une part pour déterminer dans ledit modèle (M) un contourage (C) dudit organe et une grille de cibles virtuelles (G), et
b) d'autre part pour déterminer dans ledit modèle (M) des cibles de traitement à atteindre (t1, t2, t3),
afin de générer un planning de traitement (P) comprenant les coordonnées desdites cibles de traitement (t1, t2, t3) dans le référentiel (ref 40) de ladite sonde d'imagerie (40),
- un calculateur (33) configuré pour calculer à partir dudit planning (P) les coordonnées, dans le référentiel dudit robot de guidage (ref_20), desdites cibles de traitement (t1, t2, t3) afin de générer lesdites instructions de guidage (ORD) pour guider la tête (11a) de l'aiguille (11) de manière à atteindre chacune desdites cibles de traitement (t1, t2, t3)
et dans lequel le robot de guidage est un robot multidirectionnel comprenant un bras articulé (21) qui permet le guidage de l'aiguille (11) en fonction d'instructions de guidage (ORD) reçues du module informatique de traitement (30), les instructions de guidage comprenant des informations concernant le déplacement et la profondeur, de l'aiguille pour atteindre les cibles de traitement (t1, t2, t3) et de manière à permettre une robotisation complète du geste de la curiethérapie et/ou de la biopsie,
**caractérisé en ce que** :
- les instructions de guidage (ORD) comprennent en outre des informations concernant l'orientation et l'inclinaison de l'aiguille pour atteindre les cibles de traitement, le bras articulé comportant six-axes de liberté de manière à permettre le déplacement de l'aiguille dans les trois directions de l'espace et le changement de l'inclinaison et l'orientation de l'aiguille grâce aux trois rotations autour de ces trois directions, et
- le calculateur (33) est configuré pour générer lesdites instructions de guidage (ORD) de manière à ce que, pour atteindre chacune des cibles de traitement (t1, t2, t3), l'aiguille (11) traverse la peau dudit patient (P) selon un unique point d'accès (α0), les instructions de guidage (ORD) générées permettant d'atteindre chacune des cibles de traitement (t1, t2, t3) selon un unique point d'accès de la peau traversé par l'aiguille, le calculateur (33) étant configuré pour déterminer les instructions de guidage (ORD) de manière à ce que, entre deux cibles de traitement, l'aiguille est retirée de l'organe sans être retirée du corps.

2. Système selon la revendication 1, dans lequel le robot de guidage multidirectionnel est redondant, à 7 degrés de liberté.

3. Système (100) selon l'une des revendications 1 ou 2, dans lequel ledit module informatique de traitement (30) comporte un circuit de dosimétrie (34) configuré pour déterminer une dose de produit à injecter (d1, d2, d3) pour chaque cible de traitement (t1, t2, t3) en fonction d'une estimation du volume de ladite au moins une tumeur, ladite estimation étant obtenue par une analyse de ladite image (I).

4. Système selon la revendication 3, la dose à déposer sur chaque cible de traitement est calculée automatiquement, suivant la dimension de la cible, l'inclinaison et l'orientation de l'aiguille et l'emplacement des autres cibles avoisinants déjà traités.

5. Système (100) selon la revendication 3 ou 4, dans lequel le circuit de dosimétrie (34) est configuré pour mettre à jour le planning de traitement (P) de manière à ce qu'une dose (d1, d2, d3) soit associée à chacune desdites cibles de traitement (t1, t2, t3).

6. Système (100) selon l'une des revendications 3 à 5, dans lequel ledit équipement porte-aiguille (10) comporte un chargeur (12) connecté à la base de ladite aiguille (11) et comprenant en son intérieur un produit destiné à être injecté dans au moins une desdites cibles de traitement (t1, t2, t3).

7. Système (100) selon la revendication 6, dans lequel ledit équipement porte-aiguille (10) comporte un mandrin (13) mobile, apte à coulisser à l'intérieur de ladite aiguille (11), configuré pour passer à travers ledit chargeur (12) de manière à acheminer mécaniquement ledit produit à injecter à travers ladite aiguille (11) jusqu'à la tête de celle-ci.

8. Système (100) selon la revendication 7, dans lequel ledit équipement porte-aiguille (10) comporte des moyens de motorisation (14) configurés pour motoriser le déplacement linéaire du mandrin (13) à l'intérieur de ladite aiguille (11).

9. Système (100) selon la revendication 8, dans lequel lesdits moyens de motorisation (14) sont synchronisés avec le robot de guidage (20) en fonction des instructions de guidage (ORD) dudit robot et de la dosimétrie définie dans le planning de traitement (P).

10. Système (100) selon l'une quelconque des revendications 3 à 9, le produit à injecter comportant une pluralité de grains radioactifs, dans lequel ledit équipement porte-aiguille (10) comporte un capteur de radiations (15), positionné à la sortie du chargeur (12), pour détecter le passage d'un grain radioactif du chargeur (12) vers l'aiguille (11).

11. Système (100) selon la revendication 10, dans lequel ledit chargeur (12) comporte un actionneur pas-à-pas configuré pour charger un grain radioactif dans ladite aiguille (11).

12. Système selon l'une des revendications 1 à 11 dans lequel le calculateur (33) est configuré pour générer lesdites instructions de guidage (ORD) de manière à ce que, pour atteindre chacune des cibles de traitement (t1, t2, t3), le robot opère une rotation de l'aiguille (11) autour de son axe longitudinal lors de l'insertion de l'aiguille dans l'organe (PR).

13. Système (100) selon l'une quelconque des revendications 1 à 12, dans lequel la base de l'aiguille (11) est instrumentée par un capteur de force (16) à six axes configuré pour détecter les contraintes exercées sur l'aiguille (11) lors par exemple de l'insertion de l'aiguille dans ledit organe.

14. Système (100) selon la revendication13, dans lequel ledit calculateur (33) est apte à corriger en temps réel les instructions de guidage (ORD) pour modifier l'orientation de l'aiguille (11) dans l'organe (PR) lorsque la somme des contraintes mesurées par ledit capteur de force (16) est supérieure à un seuil déterminé.

15. Système (100) selon la revendication 14, dans lequel le calculateur (33) est configuré pour modifier l'orientation de l'aiguille (11) de manière à ce que la somme des contraintes mesurées par ledit capteur de force (16) soit sensiblement égale à zéro.

16. Système (100) selon l'une quelconque des revendications 1 à 15, dans lequel ledit module informatique de traitement (30) comporte des moyens de détection (35) du bougé dudit organe, lesdits moyens comprenant :
- un réseau interne de géolocalisation (35a) comprenant au moins une première balise ancrée sur ledit organe et au moins trois deuxièmes balises ancrées sur des tissus fixes dans le corps du patient ;
- un circuit de détection (35b) configuré pour déterminer la position de chacune des balises ;
- des moyens de calcul (35c) configurés pour calculer en fonction de la position de chacune des balises les variations de la distance entre la première balise (b1) et chacune des deuxièmes balises (b2, b3, b4) afin de détecter le bougé dudit organe, lors par exemple d'une insertion (i1, i2, i3) d'une aiguille (11) dans ledit organe (PR).

17. Système (100) selon la revendication 16, dans lequel les premières (b1) et deuxièmes (b2, b3, b4) balises comportent une puce RFID.

18. Système (100) selon l'une quelconque des revendications 1 à 17, comportant un support (40), destiné à recevoir ledit patient, sur lequel est fixée ladite sonde d'imagerie (41), ledit support (40) étant un élément indépendant et détaché du robot de guidage multidirectionnel (20),
dans lequel le module informatique de traitement (30) comporte un module de calibration (36) présentant une deuxième caméra numérique (36a) fixée sur le robot de guidage (20) et une étiquette (36b) positionnée sur ledit support (40), ladite caméra (36a) étant configurée pour détecter ladite étiquette (36b) sur ledit support (40) afin de déterminer, dans le référentiel du robot, la position relative de ladite sonde d'imagerie (41) par rapport au robot de guidage (20).

19. Procédé de calibration d'un système de guidage (100) autonome d'un équipement porte-aiguille (10) selon la revendication 18, préalablement à un traitement interne d'au moins une tumeur localisée sur un organe (PR), ledit équipement (10) étant monté sur un robot de guidage (20) apte à recevoir des instructions de guidage (ORD) pour réaliser au moins une insertion (i1, i2, i3) d'une aiguille (11) dans ledit organe, ledit procédé comportant une première phase au cours de laquelle le module de calibration (36) détermine la position et l'orientation de l'aiguille (11) dans le référentiel (ref_20) du robot de guidage, et une deuxième phase au cours de laquelle le module de calibration (36) détermine la position et l'orientation de la sonde d'imagerie (40) dans le référentiel (ref_40) du robot de guidage.

20. Procédé selon la revendication 19, dans lequel la première phase comporte les étapes suivantes :
- une étape d'éclairage au cours de laquelle une source lumineuse (36e) du type laser en ligne émet en direction d'un panneau fixe ;
- une étape de déplacement au cours de laquelle ladite aiguille (11) est déplacée par le robot (20) dans le champ entre ladite source (36e) et ledit panneau selon un mouvement translatif de manière à couper le plan (PL) formé par ladite source ;
- une étape de détermination au cours de laquelle on détermine la position et l'orientation de l'aiguille (11) dans le référentiel du robot de guidage en détectant sur le panneau, à l'aide d'une deuxième caméra numérique (36d), l'intersection de l'aiguille avec le plan formé par ladite source.

21. Procédé selon la revendication 19 ou 20, ledit système comportant un support (40) destiné à recevoir ledit patient sur lequel est fixée ladite sonde d'imagerie (41), dans lequel la deuxième phase du procédé de calibration comporte une étape de détection au cours de laquelle une première caméra numérique (36a) détecte une étiquette (36b) positionnée sur ledit support (40) afin de déterminer, dans le référentiel du robot, la position relative de ladite sonde d'imagerie (41) par rapport au robot de guidage (20).

## Patentansprüche

1. Autonomes Führungssystem (100) für eine Nadelhalteausrüstung (10) zur inneren Behandlung mindestens eines Tumors auf einem Organ (PR), wobei die besagte Behandlung mindestens eine Einführung einer Nadel (11) in das besagte Organ (PR) einschließt,
bei welchem die besagte Ausrüstung (10) auf einem mehrdirektionalen Führungsroboter (20), welcher zum Empfangen von Führungsbefehlen (ORD) ausgelegt ist, angebracht ist,
wobei das besagte System (100) ein Behandlungsrechnermodul (30) aufweist, welches umfasst:
- eine zur Erzeugung eines Datenmodells (M) des Organs ausgehend von mindestens einem von einer Bildgebungssonde (41) bereitgestellten Bild (I) des besagten Organs (PR) ausgelegte Modellierungsschaltung (31),
- eine Bildbearbeitungsschaltung (32), die eingerichtet ist, um:
a) einerseits in dem besagten Modell (M) eine Kontur (C) des besagten Organs und ein virtuelles Zielgitter (B) zu ermitteln, und
b) andererseits in dem besagten Modell (M) zu erreichende Behandlungsziele (t1, t2, t3) zu bestimmen,
um einen Behandlungsplan (P) zu erzeugen, welcher die Koordinaten der besagten Behandlungsziele (t1, t2, t3) in dem Referenzsystem (ref_40) der besagten Bildgebungssonde (40) enthält,
- einen Rechner (33), welcher zum Berechnen der Koordinaten in dem Referenzsystem des besagten Führungsroboters (ref_20) der besagten Behandlungsziele (t1, t2, t3) aus dem besagten Plan (P) eingerichtet ist, um die besagten Führungsbefehle (ORD) zum Führen des Kopfes (11a) der Nadel (11) derart zu erzeugen, um jedes der besagten Behandlungsziele (t1, t2, t3) zu erreichen
und bei welchem der Führungsroboter ein mehrdirektionaler Roboter ist, welcher einen Gelenkarm (21) umfasst, der die Führung der Nadel (11) als Funktion der vom Behandlungsrechnermodul (30) empfangenen Führungsbefehle (ORD) ermöglicht, wobei die Führungsbefehle Daten betreffend die Verschiebung und die Tiefe der Nadel zum Erreichen der Behandlungsziele (t1, t2, t3) umfassen und um eine vollständige Roboterisierung der Durchführung der Brachytherapie und/oder der Biopsie zu ermöglichen, umfassen,
**dadurch gekennzeichnet, dass**:
- die Führungsbefehle (ORD) ferner Daten betreffend die Ausrichtung und Neigung der Nadel zum Erreichen der Behandlungsziele beinhalten, wobei der Gelenkarm sechs Freiheitsgrade umfasst, um die Verschiebung der Nadel in drei Richtungen des Raumes und die Veränderung der Neigung und Ausrichtung der Nadel dank dreier Drehungen um seine drei Richtungen zu ermöglichen, und
- der Rechner (33) eingerichtet ist, um die besagten Führungsbefehle (ORD) derart zu erzeugen, dass, um jedes der Behandlungsziele (t1, t2, t3) zu erreichen, die Nadel (11) die Haut des besagten Patienten (P) entlang eines einzigen Zugangspunkt (a0) durchdringt, wobei die erzeugten Führungsbefehle (ORD) es ermöglichen, jedes der Behandlungsziele (t1, t2, t3) entlang eines einzigen Zugangspunkt der Hautdurchdringung mit der Nadel zu erreichen, wobei der Rechner (33) eingerichtet ist, um die Führungsbefehle (ORD) derart zu bestimmen, dass, zwischen zwei Behandlungszielen, die Nadel aus dem Organ gezogen wird, ohne aus dem Körper gezogen zu werden.

2. System nach Patentanspruch 1, bei dem der mehrdirektionale Führungsroboter redundant ist, bei sieben Freiheitsgraden.

3. System (100) gemäß einem der Patentansprüche 1 oder 2, bei dem das besagte Behandlungsrechnermodul (30) eine Dosimetrieschaltung (34) umfasst, welche ausgelegt ist, um eine Dosis des zu injizierenden Produkts (d1, d2, d3) für jedes Behandlungsziel (t1, t2, t3) als Funktion einer Abschätzung des Volumens des besagten mindestens einen Tumors zu bestimmen, wobei die besagte Abschätzung mittels einer Auswertung des besagten Bildes (I) erhalten wird.

4. System nach dem Patentanspruch 3, die abzugebende Dosis an jedem Behandlungsziel wird automatisch berechnet, entsprechend der Größe des Ziels, der Neigung und der Ausrichtung der Nadel und der Lage der anderen bereits behandelten benachbarten Ziele.

5. System (100) gemäß den Patentansprüchen 3 oder 4, bei welchem die Dosimetrieschaltung (34) eingerichtet ist, um den Behandlungsplan (P) derart zu aktualisieren, dass eine Dosis (d1, d2, d3) jedem der besagten Behandlungsziele (t1, t2, t3) zugeordnet ist.

6. System (100) gemäß einem der Patentansprüche 3 bis 5, bei welchem die besagte Nadelhalteausrüstung (10) eine Ladevorrichtung (12) umfasst, welche an den Sockel der besagten Nadel (11) angeschlossen ist und welche in ihrem Innern ein zum Injizieren in mindestens eines der besagten Behandlungsziele (t1, t2, t2) vorgesehenes Produkt enthält.

7. System (100) gemäß dem Patentanspruch 6, bei welchem die besagte Nadelhalteausrüstung (10) ein zum Verschieben im Innern der besagten Nadel (11) geeignetes bewegliches Futter (13) umfasst, welches eingerichtet ist, um durch die besagte Ladevorrichtung (12) derart hindurchzutreten, um das durch die besagte Nadel (11) zu injizierende Produkt mechanisch bis zum Kopf derselben zu leiten.

8. System (100) gemäß dem Patentanspruch 7, bei welchem die besagte Nadelhalteausrüstung (10) Antriebsmittel (14) umfasst, welche eingerichtet sind, um die lineare Verschiebung des Futters (10) im Innern der besagten Nadel (11) anzutreiben.

9. System (100) gemäß dem Patentanspruch 8, bei welchem die besagten Antriebsmittel (14) mit dem besagten Führungsroboter (20) als Funktion der Führungsbefehle (ORD) des besagten Roboters und der in dem Behandlungsplan (P) festgelegten Dosimetrie synchronisiert sind.

10. System (100) gemäß einem der Patentansprüche 3 bis 9, wobei das zu injizierende Produkt eine Vielzahl von radioaktiven Körnern umfasst, bei welchem die besagte Nadelhalteausrüstung (10) einen Strahlungsaufnehmer (15) umfasst, welcher am Ausgang der Ladevorrichtung (12) angeordnet ist, um den Durchtritt eines radioaktiven Korns aus der Ladevorrichtung (12) zur Nadel (11) zu detektieren.

11. System (100) gemäß dem Patentanspruch 10, bei welchem die besagte Ladevorrichtung (12) einen Schritt-für-Schritt-Aktuator umfasst, welcher eingerichtet ist, um ein radioaktives Korn in die besagte Nadel (11) zu laden.

12. System gemäß einem der Patentansprüche 1 bis 11, bei welchem der Rechner (33) eingerichtet ist, um die besagten Führungsbefehle (ORD) derart zu erzeugen, dass, zum Erreichen jedes der Behandlungsziele (t1, t2, t3), der Roboter eine Drehung der Nadel (11) um seine Längsachse während des Einbringens der Nadel in das Organ (PR) durchführt.

13. System (100) gemäß einem der Patentansprüche 1 bis 12, bei welchem der Sockel der Nadel (11) mit einem Kraftaufnehmer (16) mit sechs Achsen ausgestattet ist, um die auf die Nadel (11) beispielsweise während des Einbringens der Nadel in das besagte Organ ausgeübten Dehnungen zu detektieren.

14. System (100) gemäß dem Patentanspruch 13, bei welchem der besagte Rechner (13) zum Korrigieren in Echtzeit der Führungsbefehle (ORD) geeignet ist, um die Ausrichtung der Nadel (11) in dem Organ (PR) zu verändern, wenn die Summe der von dem besagten Kraftaufnehmer (16) gemessenen Dehnungen oberhalb einer vorgegebenen Schwelle liegt.

15. System (100) gemäß dem Patentanspruch 14, bei welchem der Rechner (33) eingerichtet ist, um die Ausrichtung der Nadel (11) derart zu verändern, dass die Summe der von dem besagten Kraftaufnehmer (16) gemessenen Dehnungen im Wesentlichen gleich Null ist.

16. System (100) gemäß einem der Patentansprüche 1 bis 15, bei welchem das besagte Behandlungsrechnermodul (30) Detektionsmittel (35) von Bewegungen des besagten Organs umfasst, wobei die besagten Mittel umfassen:
- ein internes Geolokalisierungsnetz (35a) umfassend mindestens eine auf dem besagten Organ verankerte erste Markierung und mindestens drei zweite auf festen Geweben des Körpers des Patienten verankerte Markierungen;
- eine Detektionsschaltung (35b), welche zum Ermitteln der Lage jeder der Markierungen eingerichtet ist;
- Berechnungsmittel (35c), welche zum Berechnen der Variationen des Abstands zwischen der ersten Markierung (b1) und jeder der zweiten Markierungen (b2, b3, b4) als Funktion der Lage jeder der Markierungen eingerichtet ist, um die Bewegung des besagten Organs zu detektieren, beispielsweise während einer Einführung (i1, i2, i3) einer Nadel (11) in das besagte Organ (PR).

17. System (100) gemäß dem Patentanspruch 16, bei welchem die ersten (b1) und zweiten (b2, b3, b4) Markierungen einen RFID Chip umfassen.

18. System (100) gemäß einem der Patentansprüche 1 bis 17, umfassend einen Träger (40), der zum Aufnehmen des besagten Patienten vorgesehen ist, auf welchem die besagte Bildgebungssonde (41) befestigt ist, wobei der besagte Träger (40) ein unabhängiges Bauteil ist und getrennt von dem mehrdirektionalen Führungsroboter (20) ist,
bei welchem das Behandlungsrechner-Modul (30) ein Kalibrationsmodul (36) umfasst, aufweisend eine zweite Digitalkamera (36a), welche an dem Führungsroboter (20) befestigt ist, und ein auf dem besagten Träger (40) angebrachtes Etikett (36b), wobei die besagte Kamera (36a) zum Detektieren des besagten Etiketts (36b) auf dem besagten Träger (40) eingerichtet ist, um, im Referenzsystem des Roboters, die relative Lage der besagten Bildgebungssonde (41) im Verhältnis zum Führungsroboter (20) zu ermitteln.

19. Verfahren zur Kalibrierung eines autonomen Führungssystems (100) für eine Nadelausrüstung (10) gemäß Patentanspruch 18, im Vorwege einer internen Behandlung mindestens eines auf einem Organ (PR) lokalisierten Tumors, wobei die besagte Ausrüstung (10) auf einem zum Empfangen von Führungsbefehlen (ORD) zur Durchführung mindestens einer Einführung (i1, i2, i3) einer Nadel (11) in das besagte Organ geeigneten Führungsroboter (20) montiert ist,
wobei das besagte Verfahren eine erste Phase, während derer das Kalibrationsmodul (36) die Lage und Ausrichtung der Nadel (11) in dem Bezugssystem (ref _ 20) des Führungsroboters ermittelt, und eine zweite Phase, während derer das Kalibrationsmodul (36) die Lage und Ausrichtung der Bildgebungssonde (40) in dem Bezugssystem (ref _ 40) des Führungsroboters ermittelt, umfasst.

20. Verfahren gemäß dem Patentanspruch 19, bei welchem die erste Phase die folgenden Schritte umfasst:
- einen Beleuchtungsschritt, während dessen eine Lichtquelle (36e) vom Typ ausgerichteter Laser in Richtung einer festen Platte emittiert;
- einen Verschiebungsschritt, während dessen die besagte Nadel (11) durch den Roboter (20) in dem Feld zwischen der besagten Quelle (36e) und der besagten Platte entlang einer translatorischen Bewegung verschoben wird, um die von der besagten Quelle gebildete Ebene (PL) zu schneiden;
- einen Ermittlungsschritt, während dessen man die Lage und Ausrichtung der Nadel (11) im Referenzsystem des Führungsroboters ermittelt, indem man auf der Platte, mithilfe einer zweiten Digitalkamera (36d), den Schnittpunkt der Nadel mit der von der besagten Quelle gebildeten Ebene detektiert.

21. Verfahren gemäß dem Patentanspruch 19 oder 20, wobei das besagte System einen zum Aufnehmen des besagten Patienten vorgesehenen Träger (40), auf welchem die besagte Bildgebungssonde (41) befestigt ist, umfasst, bei welchem die zweite Phase des Verfahrens zur Kalibration einen Detektionsschritt umfasst, während dessen eine erste Digitalkamera (36a) ein auf dem besagten Träger (40) angebrachtes Etikett (36b) detektiert, um, in dem Referenzsystem des Roboters, die relative Lage der besagten Bildgebungssonde (41) relativ zu dem Führungsroboter (20) zu bestimmen.

## Claims

1. Autonomous guidance system (100) for a needle-holding equipment (10) for an internal treatment of at least one tumour localised on an organ (PR), said treatment involving at least one insertion of a needle (11) in said organ (PR),
wherein said equipment (10) is mounted on a multidirectional guidance robot (20) capable of receiving guidance instructions (ORD),
wherein said system (100) comprises a computerised processing module (30) comprising:
- a modelling circuit (31) configured to generate a digital model (M) of the organ from at least one image (I) of said organ (PR) provided by an imaging probe (41),
- an image processing circuit (32) configured:
a) on the one hand, to determine in said model (M) an outline processing (C) of said organ and a virtual target grille (G), and
b) on the other hand, to determine in said model (M) treatment targets to be reached (t1, t2, t3),
in order to generate a treatment plan (P) comprising the coordinates of said treatment targets (t1, t2, t3) in the reference frame (ref_40) of said imaging probe (40),
- a calculator (33) configured to calculate the coordinates from said plan (P), in the reference frame of said guidance robot (ref_20), of said treatment targets (t1, t2, t3) in order to generate said guidance instructions (ORD) to guide the head (11a) of the needle (11) so as to reach each of said treatment targets (t1, t2, t3),
and wherein the guidance robot is a multidirectional robot comprising an articulated arm (21) which makes it possible to guide the needle (11) according to guidance instructions (ORD) received from the computerised processing module (30), the guidance instructions comprising information relating to the movement and the depth, of the needle to reach the treatment targets (t1, t2, t3) and so as to make it possible for a complete robotisation of the movement of the brachytherapy and/or of the biopsy,
**characterised in that**:
- the guidance instructions (ORD) further comprise information relating to the orientation and the inclination of the needle to reach the treatment targets, the articulated arm comprising six-axes of freedom so as to make possible the movement of the needle in the three directions of space and the change of the inclination and the orientation of the needle thanks to the three rotations around these three directions, and
- the calculator (33) is configured to generate said guidance instructions (ORD) such that, to each reach the treatment targets (t1, t2, t3), the needle (11) passes through the skin of said patient (P) according to a single access point (α0), the guidance instructions (ORD) generated making it possible to reach each of the treatment targets (t1, t2, t3) according to a single access point of the skin passed through by the needle, the calculator (33) being configured to determine the guidance instructions (ORD) such that, between two treatment targets, the needle is removed from the organ without being removed from the body.

2. System according to claim 1, wherein the multidirectional guidance robot is redundant, at 7 degrees of freedom.

3. System (100) according to one of claims 1 or 2, wherein said computerised processing module (30) comprises a dosimetry circuit (34) configured to determine a product dose to be injected (d1, d2, d3) for each treatment target (t1, t2, t3) according to an estimation of the volume of said at least one tumour, said estimation being obtained by an analysis of said image (I).

4. System according to claim 3, the dose to be deposited on each treatment target is calculated automatically, according to the dimension of the target, the inclination and the orientation of the needle and the positioning of the other nearby targets already treated.

5. System (100) according to claim 3 or 4, wherein the dosimetry circuit (34) is configured to update the treatment plan (P) such that a dose (d1, d2, d3) is associated with each of said treatment targets (t1, t2, t3).

6. System (100) according to one of claims 3 to 5, wherein said needle-holding equipment (10) comprises a charger (12) connected to the base of said needle (11) and comprising therein a product intended to be injected in at least one of said treatment targets (t1, t2, t3).

7. System (100) according to claim 6, wherein said needle-holding equipment (10) comprises a mobile mandrel (13), capable of sliding inside said needle (11), configured to pass through said charge (12) so as to mechanically convey said product to be injected through said needle (11) to the head thereof.

8. System (100) according to claim 7, wherein said needle-holding equipment (10) comprises motorisation means (14) configured to motorise the linear movement of the mandrel (13) inside said needle (11).

9. System (100) according to claim 8, wherein said motorisation means (14) are synchronised with the guidance robot (20) according to the guidance instructions (ORD) of said robot and of the dosimetry defined in the treatment plan (P).

10. System (100) according to any one of claims 3 to 9, the product to be injected comprising a plurality of radioactive seeds, wherein said needle-holding equipment (10) comprises a radiation sensor (15), positioned at the output of the charger (12), to detect the passage of a radioactive seed from the charger (12) to the needle (11).

11. System (100) according to claim 10, wherein said charger (12) comprises a step-to-step actuator configured to charge a radioactive seed in said needle (11).

12. System according to one of claims 1 to 11, wherein the calculator (33) is configured to generate said guidance instructions (ORD) such that, to reach each of the treatment targets (t1, t2, t3), the robot performs a rotation of the needle (11) about the longitudinal axis thereof during the insertion of the needle in the organ (PR).

13. System (100) according to any one of claims 1 to 12, wherein the base of the needle (11) is instrumented by a force sensor (16) with six axes configured to detect the stresses exerted on the needle (11) during, for example, the insertion of the needle in said organ.

14. System (100) according to claim 13, wherein said calculator (33) is capable of correcting, in real time, the guidance instructions (ORD) to modify the orientation of the needle (11) in the organ (PR) when the sum of the stresses measured by said force sensor (16) is greater than a determined threshold.

15. System (100) according to claim 14, wherein the calculator (33) is configured to modify the orientation of the needle (11) such that the sum of the stresses measured by said force sensor (16) is substantially equal to zero.

16. System (100) according to any one of claims 1 to 15, wherein said computerised processing module (30) comprises means for detecting (35) the movement of said organ, said means comprising:
- an internal geolocation network (35a) comprising at least one first tag anchored on said organ and at least three second tags anchored on tissues fixed in the body of the patient;
- a detection circuit (35b) configured to determine the position of each of the tags;
- calculation means (35c) configured to calculate, according to the position of each of the tags, the variations of the distance between the first tag (b1) and each of the second tags (b2, b3, b4) in order to detect the movement of said organ, during, for example, an insertion (i1, i2, i3) of a needle (11) in said organ (PR).

17. System (100) according to claim 16, wherein the first (b1) and second (b2, b3, b4) tags comprise an RFID chip.

18. System (100) according to any one of claims 1 to 17, comprising a support (40), intended to receive said patient, on which is fixed said imaging probe (41), said support (40) being an element which is independent and detached from the multidirectional guidance robot (20),
wherein the computerised processing module (30) comprises a calibration module (36) having a second digital camera (36a) fixed on the guidance robot (20) and a label (36b) positioned on said support (40), said camera (36a) being configured to detect said label (36b) on said support (40) in order to determine, in the reference frame of the robot, the relative position of said imaging probe (41) with respect to the guidance robot (20).

19. Method for calibrating an autonomous guidance system (100) for a needle-holding equipment (10) according to claim 18, prior to an internal treatment of at least one tumour localised on an organ (PR), said equipment (10) being mounted on a guidance robot (20) capable of receiving guidance instructions (ORD) for carrying out at least one insertion (i1, i2, i3) of a needle (11) in said organ, said method comprising a first phase during which the calibration module (36) determines the position and the orientation of the needle (11) in the reference frame (ref_20) of the guidance robot, and a second phase during which the calibration module (36) determines the position and the orientation of the imaging probe (40) in the reference frame (ref_40) of the guidance robot.

20. Method according to claim 19, wherein the first phase comprises the following steps:
- a lighting step during which a light source (36e) of the online laser type emits in the direction of a fixed panel;
- a movement step during which said needle (11) is moved by the robot (20) in the field between said source (36e) and said panel according to a translative movement so as to cut through the plane (PL) formed by said source;
- a determination step during which the position and the orientation of the needle (11) is determined in the reference frame of the guidance robot by detecting on the panel, using a second digital camera (36d), the intersection of the needle with the plane formed by said source.

21. Method according to claim 19 or 20, said system comprising a support (40) intended to receive said patient on which is fixed said imaging probe (41), wherein the second phase of the calibration method comprises a detection step, during which a first digital camera (36a) detects a label (36b) positioned on said support (40) in order to determine, in the reference frame of the robot, the relative position of said imaging probe (41) with respect to the guidance robot (20).
